# EUROPEAN PATENT APPLICATION

(11) **EP 4 699 526 A1**
(43) Date of publication of application: **25.02.2026**
(21) Application number: 23925922.9
(22) Date of filing: 20.07.2023
(51) Int. Cl.: A61B 5/03

(54) **ARTIFICIAL INTELLIGENCE-ASSISTED INTRACRANIAL MONITORING SYSTEM AND BOUGIE ASSEMBLY**

(30) Priority: 09.03.2023 CN 202310223120
(71) Applicant: Primanova Lab (Shenzhen) Limited, Shenzhen, Guangdong 518118 (CN)
(72) Inventor: WEN, Ping, Shenzhen, Guangdong 518118 (CN)
(74) Representative: Metida
(86) International application number: PCT/CN2023/108432
(87) International publication number: WO 2024/183217

(57) **Abstract**

The present application relates to an ICM system and a probe assembly. The probe assembly includes a catheter with a front end configured to insert into the skull of a monitored person; and an FPC-sensor integrated probe sealing arranged within the catheter, the FPC-sensor integrated probe comprises an absolute pressure sensor chip; a temperature sensor chip; a signal chain chip; and a strip-shaped FPC; the absolute pressure sensor chip, the temperature sensor chip and the signal chain chip are COF encapsulated in bare chips or SMT encapsulated onto the FPC; or alternatively are COB encapsulated in bare chips or SMT encapsulated onto a rigid micro circuit board which is integrated with the FPC; the absolute pressure sensor chip, the temperature sensor chip and the signal chain chip are positioned on the FPC such that their operating ambient temperatures are substantially the same.

## Description

### TECHNICAL FIELD

The present disclosure relates to the field of innovative medical devices, in particular to an innovative, artificial intelligence-assisted intracranial monitoring system and a probe assembly.

### BACKGROUND

In various types of Intracranial Monitoring (i.e., ICM) systems, the sensor - catheter (or alternatively called as probe, strip probe, etc. ) assembly is one of the core components, and this kind of sensor component is configured to be implanted into patient's skull, such as through percutaneous or completely embedded way to realize measuring, monitoring and other purposes, in circumstances where it is necessary to measure intracranial pressure, temperature and other physiological parameters.

At present, the key technologies of intracranial monitoring system are mainly possessed by major international corporations, especially, but not limited to, the core components like sensor-catheter assembly, and traditionally they usually use wired transmission to transmit data to the external equipment like monitor. In these traditional technologies, in general, the front end (i.e., the end for intracranial insertion) of the sensor - catheter assembly is arranged with a sensor which is installed inside the catheter or located at its top, with the sensor being welded to a wire, the wire extending through the catheter to the rear end of the catheter to transmit analog signals , and the analog signal will be further processed in subsequent circuits / devices and transmitted to the monitor.

CN115399746A discloses an intracranial pressure monitoring system, including: intracranial pressure sensor, intracranial pressure monitoring components, and intracranial pressure monitor; The intracranial pressure monitoring components includes a main body, a rotor wing, a locking cap and a sealing pad; outside the front end of the main body there is provided with tapping screw, the main body is provided with an internal cavity extending from the front end to the rear end; the rotor wing is in detachable connection with the main body; the locking cap is detachably connected to the rear end of the main body, and the inner center of the locking cap is provided with a cylindrical inner core; the locking cap is provided with a through hole passing through the cap body to the cylindrical inner core; the sealing pad is circular; the intracranial pressure sensor includes a probe and a data line; the intracranial pressure monitor is used to generate and display the intracranial pressure data according to the data transmitted by the data line. The solution of CN115399746A reduces the volume of the imbedded component kept in the patent's head for monitoring intracranial pressure, by means of a detachable rotor wing, while reducing the possibility of collision; and reducing the risk of infection by setting the sealing pad and detachably attaching the locking cap with the cylindrical inner core to the rear end of the main body.

In CN115399746A, after drilling the skull hole, the rotor wing 22 is installed on the main body 21, the rotor wing 22 is screwed to drive the main body 21 to rotate, and the front end of the main body 21 is fixed to the skull hole of the patient's head 40 through the self-tapping thread in the front end of the main body 21. FIG. 1 of CN115399746A shows the assembled state of the intracranial pressure monitoring component. After the main body 21 is fixed, the rotor wing 22 is unloaded. After that, the probe of the intracranial pressure sensor 10 is implanted into the skull, and the data line 12 of the intracranial pressure sensor 10 is connected to the intracranial pressure monitor 30 through the internal cavity of the main body 21, the inner ring of the sealing pad 24, and the through-hole of the locking cap 23. The probe can transmit monitoring data to the intracranial pressure monitor 30 through the data line 12. The intracranial pressure monitor will generate and display the intracranial pressure according to monitoring data transmitted by the data line. In addition, the locking cap can be installed to the rear end of the main body 21, sealing off the internal cavity of the main body 21, thus reducing the risk of infection. FIG. 2 of CN115399746A shows the intracranial pressure monitoring system when measuring intracranial pressure, wherein the rotor wing 22 is no longer maintained in patient's head when monitoring intracranial pressure, thus reducing the risk of accidental injury.

The intracranial pressure sensor 10 and the probe disclosed in CN115399746A, whether its construction, size, or its installation mode, undoubtedly will cause great trauma to the patient's head, and cannot be used for purpose of minimally invasive surgical monitoring, at least it is not an ideal way.

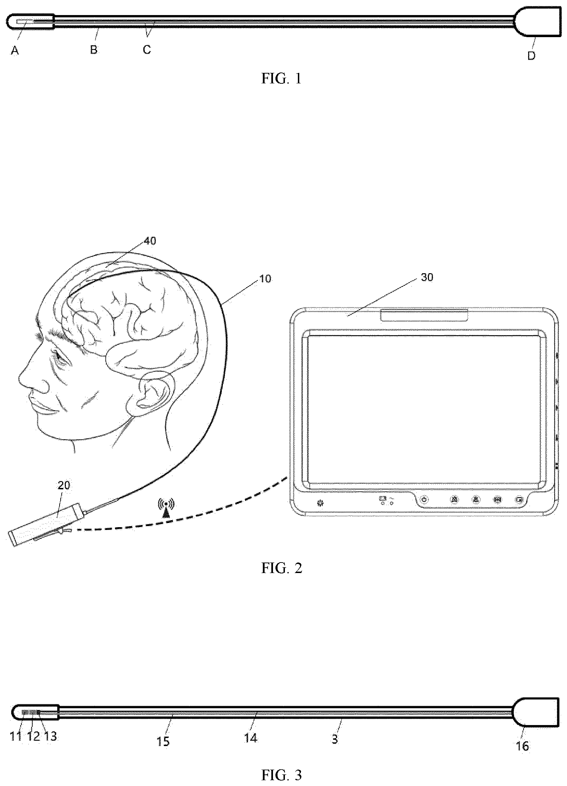

FIG. 1 schematically shows a prior-art structure of an exemplary pressure sensor - catheter for an ICM system. This type of ICM sensor - catheter generally uses gage (or absolute) pressure sensor to measure intracranial pressure, and outputs analog signal from the connector at the rear end (i.e., the right end, as shown in FIG. 1) of the catheter, the analog signal being uploaded to such as an upper device for further processing like amplification, filtering, analog-to-digital (A/D) conversion, etc. As shown in FIG. 1, in the structure of this ICM probe, the sensor A, as inserted in the patent's head in the form of a packaged device and being soldered to the wire C at the front end, is placed into the catheter B, the wire C extending all the way therethrough inside the catheter B and being connected at the rear end with a connector D such as in the form of a golden finger, such that the analog signal from the sensor device A will transmit via the wire C to the rear end, and then through the connector D to the subsequent signal processing circuit or an upper device for processing. The traditional ICM sensor - catheter assembly as shown in FIG. 1 has many defects and shortcomings, such as too large device/assembly size, enlarged surgery trauma in surgery, defective structure, process complexity, lower reliability, poor measurement precision and EMC performance, at least in but not limited to the following aspects (other aspects will be discussed later):
(i) its sensor is located inside the neurocranium when in use, while the signal processing circuit is located outside the neurocranium, and there is a significant temperature difference between the two operating environments. Considering that the temperature (temperature differences) significantly affects the sensor performance and the signal chain circuit measurement performance, it is necessary to compensate the sensor and the back-end signal chain circuit respectively. For example, the sensor temperature compensation algorithm and the signal chain chip temperature compensation algorithm are established respectively. The sensor temperature and signal chain circuit temperature are measured respectively during use, and the compensations are made through the algorithm relationship established during production, which seriously and adversely affects the production efficiency and user experience;
(ii) the sensors are configured to transmit analog signals through wires, and both the sensors and the wires are not specially processed for EMC performance, thus susceptible to parasitic capacitance, external electromagnetic environment factors, and meanwhile its connector also suffers the connection instability problem which may interfere with the analog signal, causing analog signal distortion and inaccurate measurement; and
(iii) the rear end of the probe is set to output analog signal through a soldered connector, with defects that the rear end of the probe is relatively thick, oversized and complex, and it is difficult to output the signal through wireless way since the wireless reception and transmission of the analog signal is similar to the old TV which need to adjust the antenna for proper signal reception and transmission, thus it is impossible to meet the real-time and stability of signal transmission in medical monitoring application.

Therefore, as one of the core components of the ICM system, the existing ICM probe, with traditional structure and analog signal output, can not meet the current requirement, like smaller surgical incision, more reliable structure, simpler production and process steps, higher and more stable measurement accuracy, more fidelity and more comprehensive data output, safer and more convenient use, and higher reliability.

In addition, wireless signal transmission is the current development trend of modern medical monitoring sensors and catheters, which can make it more convenient for medical staff to use and reduce various risks in the process of use.

Therefore, it is desirable in the present field to develop an improved intracranial monitoring (ICM) system and its innovative probe assembly, in order to reduce or even overcome the defects in the existing technology, and to achieve more beneficial effects and technology progress.

The information included in this section of the specification of the present disclosure, including any references referenced herein and any descriptions or discussions thereof, are incorporated only for the purpose of reference, thus shall not to be considered as limitation to the scope of the present disclosure.

### SUMMARY

In view of the above and other more concepts, the present disclosure is proposed.

According to one aspect of the present disclosure, it is intended to provide a probe assembly for an intracranial monitoring (ICM) system, the probe assembly including: a catheter having a hollow cavity extending between a front end and a rear end of the catheter, the front end being configured to insert into the skull of a monitored person; and an FPC(i.e., Flexible Printed Circuit)-sensor integrated probe sealing arranged within the hollow cavity of the catheter, wherein the FPC-sensor integrated probe comprises: an absolute pressure sensor chip arranged for monitoring of intracranial pressure of the monitored person; a temperature sensor chip arranged for monitoring intracranial temperature of the monitored person; a signal chain chip; and a strip-shaped flexible printed circuit (i.e., FPC); wherein the absolute pressure sensor chip, the temperature sensor chip, or the signal chain chip are COF encapsulated in bare chips or SMT encapsulated onto the flexible printed circuit; or alternatively, the absolute pressure sensor chip, the temperature sensor and/or the signal chain chip are COB encapsulated in bare chips or SMT encapsulated onto a micro circuit board in the form of a mini rigid substrate, and the micro circuit board is integrated and electrically connected with the flexible printed circuit; wherein the absolute pressure sensor chip, the temperature sensor chip and the signal chain chip are positioned on the flexible printed circuit such that their operating ambient temperatures are substantially the same; wherein the flexible printed circuit is designed to further integrate a power line and a signal transmission line, such that the flexible printed circuit has power transmission and signal transmission function; and wherein the flexible printed circuit is electromagnetically compatible (i.e., EMC).

According to an embodiment, the absolute pressure sensor chip, the temperature sensor chip, and the signal chain chip are integrated and encapsulated on an end of the flexible printed circuit which is located at the front end of the catheter; or alternatively, the absolute pressure sensor chip, the temperature sensor chip and the signal chain chip are integrated and encapsulated on the micro circuit board, wherein the micro circuit board is integrated and electrically connected on an end of the flexible printed circuit which is located at the front end of the catheter.

According to an embodiment, the probe assembly is constructed such that the absolute pressure sensor can protrude from the front end of the catheter, and preferably the absolute pressure sensor may be covered by a protective cover.

According to an embodiment, the signal transmission line is a digital signal transmission line designed to transmit digital signal.

According to an embodiment, the absolute pressure sensor chip, the temperature sensor chip and the signal chain chip are sequentially arranged on the flexible printed circuit, along a lengthwise direction of the Flexible Printed Circuit from the front end to the rear end.

According to an embodiment, the absolute pressure sensor chip and/or the temperature sensor chip and/or the signal chain chip are COF encapsulated onto the flexible printed circuit by means of die Bonding and wire Bonding of bare chips; or alternatively, the absolute pressure sensor chip and/or the temperature sensor chip and/or the signal chain chip are COB encapsulated onto the micro circuit board by means of die Bonding and wire Bonding of bare chips.

According to an embodiment, the intracranial monitoring system is an artificial intelligence assisted intracranial monitoring system, and wherein the catheter is a neurophysiological monitoring catheter.

According to an embodiment, the probe assembly further comprises a rear-end circuit which is integrated or alternatively connected to a rear end of the flexible printed circuit through a connector, the rear-end circuit comprising at least one of a filter circuit, an amplification circuit, a A/D converter and a D/A converter.

According to an embodiment, the rear-end circuit further comprises an integrated wireless communication chip.

According to an embodiment, the catheter is used for neurophysiological monitoring.

According to an embodiment, the minimum spacings between the absolute pressure sensor chip and the signal chain chip, and between the temperature sensor chip and the signal chain chip, are respectively no greater than 2 mm, such as no greater than 0.5 mm.

According to an embodiment, the width of the strip-shaped flexible printed circuit is less than 3 mm, such as in the range of 1 to 2 mm, or preferably no greater than 0.8 mm.

According to an embodiment, the width of the strip-shaped flexible printed circuit is less than 0.8 mm.

According to an embodiment, the micro circuit board in the form of a mini rigid substrate is electrically connected with the flexible printed circuit through conductive adhesive.

According to an embodiment, the area of the absolute pressure sensor chip is less than 2 square millimeters, for example, less than 1 square millimeter.

According to an embodiment, the length of the strip-shaped Flexible Printed Circuit is more than 80 mm, for example more than 200 mm.

According to an embodiment, the length of the strip-shaped Flexible Printed Circuit is in the range of 400 to 1500 mm.

According to an embodiment, the flexible printed circuit is a single-sided circuit board or a double-sided flexible circuit board, and wherein on either side of the flexible printed circuit there is provided with an electromagnetic shielding layer.

According to an embodiment, the flexible printed circuit is a double-sided flexible circuit board processed based on a flexible copper clad laminate, wherein the absolute pressure sensor chip, the temperature sensor chip, the signal chain chip and the signal transmission line are arranged on a front side of the double-sided flexible circuit board, and the power line is arranged on an opposite back side of the double-sided flexible circuit board.

According to an embodiment, the electromagnetic shielding layer is selected from at least one of the following: a silver foil, a copper foil, a silver-containing coating, and a copper coating.

According to another aspect of the present disclosure, there is provided an artificial intelligence assisted intracranial monitoring system, wherein the artificial intelligence auxiliary intracranial monitoring system comprises: the probe assembly as described above, and a monitor, the monitor comprising: a display for displaying monitored parameters; a pair of wireless communication modules configured to perform wireless digital communication between the probe assembly and the monitor, which comprises a first wireless communication module arranged on the probe assembly, and a second wireless communication module arranged on the monitor in pairing and in encrypted communication with the first wireless communication module; and a host computer, which comprises at least a mainboard with a CPU, and an artificial intelligence-assisted prediction module integrated in the mainboard.

According to an embodiment, the artificial intelligence-assisted intracranial monitoring system is configured to directly use the sensed data of the temperature sensor to perform temperature compensation calibration of a digital output signal of the probe assembly.

According to an embodiment, the digital output signal goes through only one temperature compensation calibration before transmission to monitor.

According to an embodiment, the artificial intelligence-assisted intracranial monitoring system further comprises accessories, the accessories including at least one of a multifunctional adapter, a puncture needle, and a subcutaneous tunnel type transfixion needle.

According to an embodiment, the first wireless communication module and the second wireless communication module are Bluetooth communication modules.

According to an embodiment, the temperature sensor is a sensor in the form of a PTN (thermistor).

According to an embodiment, the temperature sensor is a PN junction temperature sensor integrated with the signal chain chip.

According to the concept in still another aspect of the present disclosure, there is provided a probe assembly for an intracranial monitoring (ICM) system, the probe assembly including: a catheter having a hollow cavity extending between a front end and a rear end of the catheter, the front end being configured to insert into the skull of a monitored person; and an FPC - sensor integrated probe sealing arranged within the hollow cavity of the catheter, wherein the FPC - sensor integrated probe comprises: an absolute pressure sensor chip arranged for monitoring of intracranial pressure of the monitored person; a temperature sensor chip arranged for monitoring intracranial temperature of the monitored person; a signal chain chip; and a strip-shaped flexible printed circuit; wherein the absolute pressure sensor chip, the temperature sensor chip and the signal chain chip are integrated and encapsulated onto a micro circuit board in the form of a mini rigid substrate, and the micro circuit board is integrated and electrically connected with the flexible printed circuit; wherein the absolute pressure sensor chip, the temperature sensor chip and the signal chain chip are positioned on the flexible printed circuit such that their operating ambient temperatures are substantially the same; wherein the flexible printed circuit is designed to further integrate a power line and a signal transmission line, such that the flexible printed circuit has power transmission and signal transmission function; wherein the flexible printed circuit is electromagnetic compatible; and wherein the length of the strip-shaped flexible printed circuit is equal to or more than 200 mm.

The innovative probe assembly and artificial intelligence assisted intracranial monitoring (ICM) system provided in the present disclosure overcome many inherent technical defects and disadvantages of traditional ICM systems and probes, such that the innovative ICM probe affords more flexibility in circuit design and wiring, higher structural strength, more simple process and lower cost, more consistent processing property, higher reliability, higher measurement precision, and greater stability, better EMC compatibility with greater resistance to electromagnetic interference, making it possible smaller surgical wound and thus less trauma, providing more fidelity of data output, and more convenient and reliable user experience.

In addition, the ICM system according to one or more embodiments of the present disclosure also makes it possible for the wireless signal transmission and the timely artificial intelligence-assisted prediction, which can enable the medical staff to use it more conveniently, and to further reduce the risk of the care recipient (i.e., the patient) being exposed to dangerous situations during its use. By making the signal and data transmission wireless, it can realize the wireless connection between the probe (or probe assembly) on the patient side and the monitor such as at or near bed side, thus can release or remove the restrictions of the wired connection arrangements of the existing commercial ICM system products on the patient's position and activities. Further, the present disclosure makes it possible to eliminate the probe displacement and mis-extraction of the probe assembly, so as to reduce the risk of intracranial infection, bleeding, cerebrospinal fluid leakage and other complications of the cared / monitored patient, and the ICM system can be made more intelligent and user-friendly.

Further embodiments of the present disclosure may achieve other beneficial technical effects not listed individually, which may be partially described herein below and which are apparent and understood by a person skilled in the art after reading the present disclosure.

### BRIEF DESCRIPTION OF THE DRAWINGS

The above features and advantages and other features and advantages of these embodiments, and the embodiment way thereof, would be apparent to those skilled in the art, and the present disclosure as well as the embodiments thereof may be better understood by reference to the following Figures and the related description.
FIG. 1 is a schematic view of the structure of an example of an ICM sensor-catheter of the prior art.
FIG. 2 is a schematic view of an intracranial monitoring (ICM) system according to an embodiment of the present disclosure, showing its basic configuration.
FIG. 3 is a schematic view of the overall structure of an FPC-sensor integrated probe for an ICM system according to an embodiment of the present disclosure.
FIG. 4 is a partially enlarged schematic view of the front end structure of the overall structure of the FPC-sensor integrated probe shown in FIG. 3, wherein the sensor (bare chip) is directly packaged onto the FPC.
FIG. 5 is a partially enlarged schematic view of the front end structure of the FPC-sensor integrated probe according to another embodiment of the present disclosure, wherein the sensor (bare chip) is packaged onto a micro circuit board in the form of a rigid PCB arranged on the FPC."
FIG. 6 is a schematic view of the overall structure of an FPC-sensor integrated probe for an ICM system according to yet another embodiment of the present disclosure, wherein a rear-end circuit is directly integrated at the rear end of the FPC.

### DETAILED DESCRIPTION

Details of one or more embodiments of the present disclosure will be set forth in the following description of the accompanying drawings and detailed description. Other features, objects, and advantages of the present disclosure may be apparent from these descriptions, drawings, and claims.

It should be understood that the illustrated and described embodiments are not limited in applications to the details of construction and arrangement of components set forth in the following description or illustrated in the accompanying drawings. The illustrated embodiments may be other variant embodiments and can be implemented or executed in various ways. The examples are provided by way of explanation of the disclosed embodiments and not by way of limitation. Indeed, it will be apparent to those skilled in the art that various modifications and variations can be made to the embodiments of the present disclosure without departing from the scope or spirit of the present disclosure. For example, features illustrated or described as part of one embodiment may be used with another embodiment to still produce additional embodiments. Accordingly, the present disclosure encompasses such modifications and variations that fall within the scope of the appended claims and their equivalents.

Also, it is to be understood that the wordings "comprising", " including", "having" and variations thereof used herein are for the purpose of description in an open manner and intended to contain items listed thereafter and equivalents thereof, as well as additional items if appropriate.

The present disclosure will be described in more detail below with reference to the attached drawings and several specific embodiments.

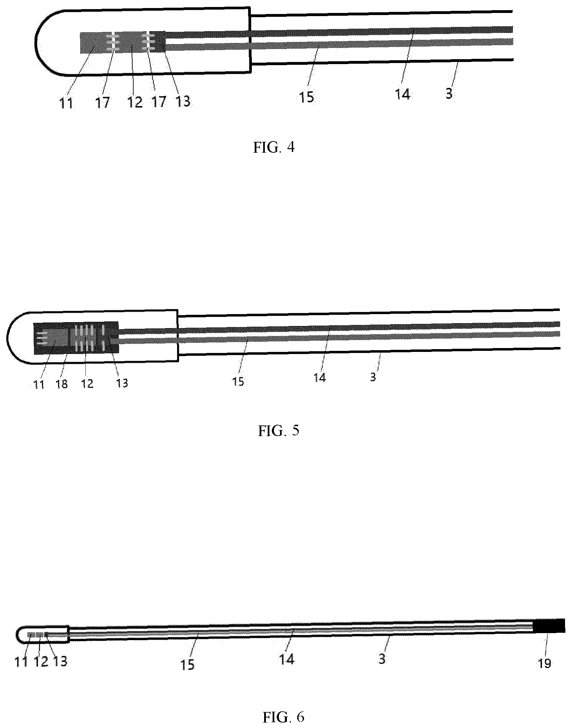

FIG. 3 and FIG. 4 schematically show an overall structure of an FPC-sensor integrated probe for an ICM system according to an embodiment of the present disclosure, wherein FIG. 4 is a partially enlarged view of the front end structure of the overall structure of the FPC-sensor integrated probe shown in FIG. 3, with multiple sensors (bare chips) being directly packaged onto the FPC (i.e., Flexible Printed Circuit).

For example, as described in Background, the probes of existing intracranial monitoring systems will measure and monitor intracranial pressure, one of the most important physiological parameters, and will generally adopt a gauge pressure sensor, which is connected to the wire by soldering, or alternatively welded to a PCB board in the form of a rigid PCB, which PCB is then soldered to one end of the wire. In this art, the used wires and PCB, due to its inherent characteristics and manufacturing process, is still of big size in its width and thickness, and with also a relatively large catheter - since the gauge pressure sensing technology require that the catheter air-passage not be narrowed or blocked, as this is relatively disadvantageous and unwanted, as these types of big or large size will lead to form a relatively large cranial wound which is undesirable.

Moreover, in the key components of the traditional ICM catheters, the data is usually transmitted to the monitor by wired transmission. The front end (i.e., the end inserted into the patent's skull) of such catheter is equipped with a sensor which is installed inside the catheter or the protective cover arranged at the tip of the catheter. The sensor is soldered to the wires, and the wires extend all the way through the catheter to its rear end to output analog signals, which will be further processed in the subsequent circuit/device and transmitted to the monitor. Traditional wires used herein generally transmit analog signals, and do not have digital signal processing capabilities, thus it is impossible to arrange thereon circuits such as digital signal processing circuits, and their robustness, reliability and electromagnetic compatibility are relatively low, which is not matched and thus incompatible with those ICM systems and applications with high performance requirements in various aspects. In the applications of current ICM systems, it is desirable for the ICM probes to have superior EMC compatibility and electromagnetic shielding performance to ensure the continuity, stability, and fidelity of data and signal output while keeping minimized size, which can not be provided through arrangement of traditional wires.

In view of the above, according to one embodiment shown in Figs. 3-4 of the present disclosure, the inventors of the present disclosure creatively propose to use the technology of Flexible Printed Circuit (FPC) instead of traditional wires as the carrier/support body of the probes, and creatively propose the overall design of an FPC-sensor integrated probe for the ICM system.

Flexible Printed Circuit (FPC) is a newly developed circuit board in recent years in a flexible and pliable form, which has been widely used in the field like semiconductors, LED lighting packages in recent years, with excellent robustness, reliability, flexibly customized circuit design and device integration capabilities, as well as attainable good electromagnetic compatibility.

Due to the development of FPC technologies, in recent years, it has become possible to fabricate Flexible Printed Circuits in the form of strips with smaller width, such as less than 3 mm, for instance in the range of 1-2 mm, and even FPC strips of less than 0.8 mm in width are possible, such as narrow FPC strips fabricated from FCCL (Flexible Copper Clad Laminate). Through study and repeated experimental verification, the inventors of the present disclosure surprisingly found that by integrating the sensor (s) required by the ICM system with the FPC into an integrated ICM probe in a monolithic design, it is not only possible to obtain an ICM probe with smaller size - and thus smaller surgical wound, but also its process steps are made simpler, with higher yield in the process, lower stress therein caused in the manufacture process, and lower cost. Besides, the key points lie in the fact that the robustness and electromagnetic compatibility and interference resistance of such integrated ICM probe will be greatly improved, and unlike traditional manufacture process which relies solely on many manual steps / production, it enables mass production and automation of key process steps (including but not limited to the packaging of sensor chips / signal chain chips), resulting in higher consistency, stability, lifetime and reliability of final products, and higher measurement accuracy thereof, which are very desirable and even critical for ICM system and its applications.

According to an example, the probe assembly which can be used in an intracranial monitoring system, may comprises: a catheter, which may have a hollow cavity extending between a front end and a rear end of the catheter, the front end being configured to insert into the skull of the monitored person. The probe assembly may also include an FPC-sensor integrated probe sealingly arranged into the hollow cavity of the catheter. The FPC-sensor integrated probe may include: an absolute pressure sensor chip for monitoring intracranial pressure; a temperature sensor chip that can be used to monitor intracranial temperature; signal chain chip; strip-shaped Flexible Printed Circuit; the absolute pressure sensor chip, the temperature sensor chip and the signal chain chip can be packaged in the form of bare chips with COF (Chip On Flexible Printed Circuit) package technology or SMT process, onto a Flexible Printed Circuit. Or alternatively , the absolute pressure sensor chip, the temperature sensor and the signal chain chip can be packaged in the form of bare chips with COF (Chip On Flexible Printed Circuit) package technology or SMT process, onto a Micro Circuit Board in the form of a mini rigid substrate, and the Micro Circuit Board in turn can be integrated and connected with a Flexible Printed Circuit (FPC), or alternatively in electrical connection with an FPC. As a non-restrictive example, the Micro Circuit Board in the form of the mini rigid substrate can be integrated and electrically connected with an FPC through printing or coating conductive adhesive, so as to minimize or eliminate adverse heat affecting of welding/soldering. The absolute pressure sensor chip, the temperature sensor chip, and the signal chain chip can be positioned on the Flexible Printed Circuit such that the temperature of the environment in which they operate is essentially the same. The Flexible Printed Circuit can be designed to further integrate power lines and signal transmission lines, so that the Flexible Printed Circuit has functions of both power transmission and signal transmission, especially digital signal transmission. As a preferred requirement for ICM system applications, Flexible Printed Circuits should have good electromagnetic compatibility (EMC) property and thus can provide good resistance to environmental electromagnetic interference.

According to an example, the absolute pressure sensor chip, the temperature sensor chip, and the signal chain chip can be COF packaged, by means of Die Bonding and Wire Bonding of bare chips, onto a Flexible Printed Circuit.

According to another example, the absolute pressure sensor chip, the temperature sensor chip, and the signal chain chip can be COF packaged, by means of Die Bonding and Wire Bonding of bare chips, onto a Micro Circuit Board in the form of a mini rigid substrate. More specifically, as shown in Figs. 3-4, according to the concept of the illustrated embodiment, it is possible that an FPC of 0.8 mm in width or even less, can be used in the FPC-sensor integrated probe of the present disclosure for ICM system. To this end, in the length extension direction of the FPC, i.e., in a direction extending longitudinally from the distal end (i.e., the end which is inserted into the patent's skull) to the proximal end of the FPC, an absolute pressure sensor chip 11, a signal chain chip 12, and a temperature sensor chip 13 are sequentially arranged, integrated and packaged onto the FPC, to provide direct measurement parameters, at least such as ICT and ICP. As an example, the absolute pressure sensor chip 11 may be exposed from the front end (i.e., the front end where the FPC being inserted intracranial) of the catheter, and preferably the absolute pressure sensor chip 11 may be covered by a protective cover to ensure reliability, stability and robustness of its operation. The area of the absolute pressure sensor chip 11 may generally be less than 2 square millimeters, for example below 1 square millimeter. Moreover, the spacing between the absolute pressure sensor chip 11 and the signal chain chip 12, as well as between the signal chain chip 12 and the temperature sensor chip 13, is generally less than 0.5 mm, so that the ambient temperature difference between them is very small and almost negligible, and such temperature difference not only affects the sensor/transducer measurement, but also affects the performance of the signal chain chip circuit. Thus, this arrangement minimizes the temperature difference, makes it possible for a single temperature compensation, and thus can improve the measurement accuracy. Although the power line 14 and the digital signal line 15 are shown to be arranged on the same side of the FPC as the sensor, etc., to provide both power and digital transmission, they can be respectively arranged on both sides of the FPC circuit board if the FPC is a double-sided flexible circuit board. For example, in the case of the FPC being a double-sided flexible circuit board, the pressure sensor, the temperature sensor chip, the signal chain chip can be arranged at the same side of the FPC, and the power line (and may other lines, such as digital signal lines, etc.) can be arranged at the other opposite side of the FPC. Moreover, those skilled in the art can understand, other appropriate arrangement of the sensors and chips are also possible and are all within the scope of the present disclosure.

Additionally, in the prior art, generally a bridge circuit is used to compensate temperature without any real physical temperature sensor. In the present disclosure, for example, a temperature sensor chip 13, in a physical form, such as an NTC, is arranged directly adjacent to the signal chain chip 12, to provide temperature measurement and a direct temperature compensation input. Of course, as an additional example, a PN junction temperature sensor can also be employed, and can be directly integrated onto the signal chain chip 12.

As shown in FIG. 3-4, these devices and chips are integrated and packaged on the FPC to provide an integrated probe, so it not only makes the digital processing, digital transmission and output of the ICM probe possible, but also eliminates the heat effect of welding on the probe and its sensor since no additional welding process is required.

The length of the FPC board can be customized freely, and is generally more than 200 mm for a ICM probe, such as in the range of 400 to 1500 mm. Further, compared with wire, because of the natural advantages of the FPC structure and properties, for example, the FPC in the form of FCCL, can be fabricated into a single-side FPC or a double-side PCB form of FPC, and can be easily provided with an electromagnetic shielding protection layer, such as silver coil, copper foil, silver or copper coating, to meet EMC requirements, and to eliminate environmental electromagnetic interference during data processing and data transmission.

FPC, whether single-sided or double-sided FPC, can easily integrate power supply circuit and signal transmission circuit because of its own circuit and wiring function. For example, in the case of the FPC being a double-sided flexible circuit board, it is possible to arrange on the front side of the FPC a absolute pressure sensor chip 11, a signal chain chip 12, a temperature sensor chip 13 and other related circuit and components, such as data transmission circuit, etc., and the power supply circuit can be arranged on the opposite side of the FPC, so that such design on the front side of the FPC can save space for circuit, this is preferred for the ICM probe where the FPC width is required to be as narrow as possible.

According to an example, the absolute pressure sensor chip, the temperature sensor chip, and the signal chain chip may be integrated onto the end of the Flexible Printed Circuit which will be placed at the front end of the catheter.

According to an example, an absolute pressure sensor chip, a signal chain chip, and a temperature sensor chip may be sequentially arranged on the Flexible Printed Circuit from its frond end to its rear end along the lengthwise direction of the Flexible Printed Circuit.

According to an example, the area of the absolute pressure sensor chip is less than 2 square millimeters, for example, less than 1 square millimeter. In general, a smaller absolute pressure sensor chip makes it technically feasible to use narrower FPC, thus reducing the size of the probe and reducing the surgical wound and trauma, which is advantageous.

According to an example, the probe assembly may further include a rear-end circuit integrated in or connected through a connector to the rear end of the Flexible Printed Circuit, and the rear-end circuit may comprise at least one of a filter circuit, an amplification circuit, an A/D converter, and a D/A converter.

According to an example, the rear-end circuit may further comprise an integrated wireless communication chip, which may be integrated with the rear-end circuit or alternatively in a wired communication with it.

According to one example, the minimum spacings between the absolute pressure sensor chip and the signal chain chip, and between the temperature sensor chip and the signal chain chip, are respectively no greater than 0.5 mm.

FIG. 5 shows another embodiment of the FPC-sensor integrated probe of the present disclosure, illustrating a partially magnified schematic view of the front end structure of the FPC-sensor integrated probe in this embodiment, wherein the sensor (in the form of bare chip) is encapsulated (packaged) onto a micro circuit board 18 in the form of a micro rigid substrate, which is in turn arranged on the FPC. Other than the embodiment shown in FIG. 3-4, in the embodiment of FIG. 5, the micro circuit board 18 in the form of a micro rigid PCB or a rigid substrate, are connected - for example through conductive adhesive or wire soldering - on the front end of the FPC; and on the micro circuit board, for example, in its lengthwise direction from the distal end to the proximal end, an absolute pressure sensor chip 11, a signal chain chip 12 and a temperature sensor chip 13 are sequentially integrated and packaged. Therefore, on one hand, the advantages of the PCB, in its rigid form, are available, such as better maintainability, stability, easy to assemble and design, smaller deformation and higher robustness; on the other hand, the convenience and flexibility of FPC in its circuit design and wiring, the weldability, the reliability and stability of its structure and strength, the EMC compatibility and other advantages, are also available.

According to an example, an absolute pressure sensor chip, a temperature sensor chip, and a signal chain chip may be packaged together on a micro circuit board, wherein the micro circuit board may be integrated and electrically connected to the end of the Flexible Printed Circuit placed at the front tip of the catheter.

In the embodiment shown in FIG. 3 , the rear end of the FPC - sensor integrated probe can be connected through a connector 16 with a rear-end circuit (and an optional wireless communication module). As another example, FIG. 6 shows a schematic view of the overall structure of another FPC-sensor integrated probe, in which a rear-end circuit 19 is directly integrated at the rear end of the FPC, eliminating the connector 16 such as in the form of a costly additional gold finger and its time-consuming installation process. A wireless communication module, such as a Bluetooth communication module, can also be integrated at the rear end of the FPC, as further described below.

FIG. 2 is a schematic view of the basic configuration of the intracranial monitoring (ICM) system according to the an embodiment of the present disclosure. As shown in FIG. 2, the intracranial monitoring (ICM) system according in this embodiment includes a new and innovative form of an FPC-sensor integrated probe 10 configured to inserted into the patient's brain tissue or ventricle 40, to provide monitoring and measurement. The front end (or head tip) of the probe 10 is shown inserted into the patient's neurocron 40, and accessories such as a handle 20 can be installed at the rear end of the probe 10. the data, in the form of digital signals, as sensed by the FPC-sensor integrated probe 10, can be transmitted to the monitor 30 through wireless digital transmission, for example, through paired wireless communication modules (not shown).

The wireless communication module can be, for example, a pair of Bluetooth communication modules, arranged for wireless digital communication between the monolithic integrated probe 10 and the monitor 30, which includes: a first Bluetooth communication module (not shown) arranged on or in circuit integration with the rear end of the FPC of the probe 10, and a paired second Bluetooth communication module (not shown), which is for example arranged on the monitor 30 or other associated devices connected thereto. The antenna of the second wireless communication module, for example, can be attached to the monitor 30 inside its housing. As an example, data transmission and communication between the first and second Bluetooth communication modules may be conducted through such as Bluetooth 5.2 version or other versions of communication protocol, and preferably in encrypted communication for data security. Bluetooth communication can provide good communication in a distance within around 20 meters, which is enough for the general application scenarios of the ICM system.

The monitor 30 may generally include a display for displaying the measured / monitored parameters, and a host computer, which host computer may, for example, be in the form of a personal computer, an industrial computer, or a cloud computer; and the host computer, as an example, may have at least a mainboard with a CPU or ECU, and optionally an AI-assisted prediction module that can be built into the mainboard CPU in the form of programs/modules - in the case of AI-assisted intracranial monitoring systems. The AI-assisted prediction module is configured to conduct real time calculation and obtain some derived prediction parameters/data according to the data from the probe 10 (and possibly some other inputs), to provide artificial intelligence (AI) assisted prediction, and real-time calculation of predictive intracranial derived parameters. Such AI-assisted prediction module makes it possible for prediction and early warning of disease risk, which can satisfy an unmet clinical need in the aspect of device function, i.e., makes it possible to 4-6 hours in advance diagnose dangerous disease prognosis in a wireless way.

According to one example, the AI-assisted intracranial monitoring (ICM) system can be configured for temperature compensation and calibration for the digital output signal of the probe assembly, by directly using the sensed data of temperature sensor chip.

According to one example, to achieve a high accuracy standard, the digital output signal only needs to go through one (i.e., a single pass) calibration temperature compensation before being transmitted to monitor.

According to one example, the artificial intelligence-assisted intracranial monitoring system may further include accessories comprising at least one of a multifunctional adapter, a puncture needle, and a subcutaneous tunnel type transfixion needle. An example of a multifunctional adapter can refer to the Chinese Utility Model "A Multifunctional Adapter" with an application No. 202221240938.7, as owned by the same applicant as the present disclosure, the content of which is incorporated herein by reference.

According to an example, the first wireless communication module and the second wireless communication module may be Bluetooth communication modules.

According to one example, the temperature sensor can be a sensor in the form of PTN (or thermistor).

According to an example, the temperature sensor may be a PN junction temperature sensor integrated in the signal chain chip.

The innovative probe assembly and the artificial intelligence-assisted intracranial monitoring system provided in the present disclosure offer a number of technical advantages over prior art, including: the ICM probe can be more flexible and more integrated in its circuit design and wiring; higher structural strength, more simple manufacturing process and lower cost, higher consistency in production process and thus product properties, higher reliability, higher measurement precision and stability, higher resistance to electromagnetic interference and thus better EMC performance, making it possible smaller surgical wound and thus less trauma to the patient, providing more fidelity of data output, and more convenient and reliable user experience. The aforesaid ICM system also makes it possible for the wireless signal transmission and the timely artificial intelligence-assisted prediction, which can enable the medical staff to use it more conveniently, and to further reduce the risk of the care recipient (i.e., the patient) being exposed to dangerous situations during its use.

The foregoing description of various embodiment of the present disclosure is presented for purposes of illustration. The foregoing description is not intended to be exhaustive or to limit the present disclosure to the precise steps and/or forms as disclosed above, obviously, many modifications and variations can be made in light of the above teachings. It is intended that the scope and its equivalents of the present disclosure will be defined by the appended claims.

## Claims

1. A probe assembly for an intracranial monitoring system, the probe assembly including: a catheter having a hollow cavity extending between a front end and a rear end of the catheter, the front end being configured to insert into the skull of a monitored person; and an FPC - sensor integrated probe sealing arranged within the hollow cavity of the catheter,
wherein the FPC - sensor integrated probe comprises:
an absolute pressure sensor chip arranged for monitoring of intracranial pressure of the monitored person;
a temperature sensor chip arranged for monitoring intracranial temperature of the monitored person;
a signal chain chip; and
a strip-shaped flexible printed circuit;
wherein the absolute pressure sensor chip, the temperature sensor chip, and/or the signal chain chip are COF encapsulated in bare chips or SMT encapsulated onto the flexible printed circuit; or alternatively, the absolute pressure sensor chip, the temperature sensor /or the signal chain chip are COB encapsulated in bare chips or SMT encapsulated onto a micro circuit board in the form of a mini rigid substrate, and the micro circuit board is integrated and electrically connected with the flexible printed circuit;
wherein the absolute pressure sensor chip, the temperature sensor chip and the signal chain chip are positioned on the flexible printed circuit such that their operating ambient temperatures are substantially the same;
wherein the flexible printed circuit is designed to further integrate a power line and a signal transmission line, such that the flexible printed circuit has power transmission and signal transmission function; and
wherein the flexible printed circuit is electromagnetically compatible.

2. The probe assembly according to claim 1, wherein the absolute pressure sensor chip, the temperature sensor chip, and the signal chain chip are integrated and encapsulated on an end of the flexible printed circuit which is located at the front end of the catheter;
or alternatively,
the absolute pressure sensor chip, the temperature sensor chip and the signal chain chip are integrated and encapsulated on the micro circuit board, wherein the micro circuit board is integrated and electrically connected on an end of the flexible printed circuit which is located at the front end of the catheter.

3. The probe assembly according to claim 2, wherein the absolute pressure sensor chip, the temperature sensor chip and the signal chain chip are sequentially arranged on the flexible printed circuit, along a lengthwise direction of the Flexible Printed Circuit from the front end to the rear end.

4. The probe assembly according to claim 1, wherein the probe assembly further comprises a rear-end circuit which is integrated or alternatively connected to a rear end of the flexible printed circuit through a connector, the rear-end circuit comprising at least one of a filter circuit, an amplification circuit, a A/D converter and a D/A converter.

5. The probe assembly according to claim 4, wherein the rear-end circuit further comprises an integrated wireless communication chip.

6. The probe assembly according to any of claims 1 to 5, wherein the minimum spacings between the absolute pressure sensor chip and the signal chain chip, and between the temperature sensor chip and the signal chain chip, are respectively no greater than 2 mm, such as no greater than 0.5 mm.

7. The probe assembly according to any of claims 1 to 5, wherein the width of the strip-shaped flexible printed circuit is less than 3 mm, such as in the range of 1 to 2 mm, or preferably no greater than 0.8 mm.

8. The probe assembly according to claim 7, wherein the width of the strip-shaped flexible printed circuit is less than 0.8 mm.

9. The probe assembly according to any of claims 1 to 5, wherein the micro circuit board in the form of a mini rigid substrate is electrically connected with the flexible printed circuit through conductive adhesive.

10. The probe assembly according to any of claims 1 to 5, wherein the probe assembly is constructed such that the absolute pressure sensor protrudes from the front end of the catheter.

11. The probe assembly according to any of claims 1 to 5, wherein the flexible printed circuit is selected from a single-sided circuit board and a double-sided flexible circuit board, and wherein on either side of the flexible printed circuit there is provided with an electromagnetic shielding layer.

12. The probe assembly according to claim 11, wherein the flexible printed circuit is a double-sided flexible circuit board fabricated from a flexible copper clad laminate, wherein the absolute pressure sensor chip, the temperature sensor chip, the signal chain chip and the signal transmission line are arranged on a front side of the double-sided flexible circuit board, and the power line is arranged on an opposite back side of the double-sided flexible circuit board.

13. The probe assembly according to claim 11, wherein the electromagnetic shielding layer is selected from at least one of the following: a silver foil, a copper foil, a silver-containing coating, and a copper coating.

14. The probe assembly according to any of claims 1 to 13, wherein the absolute pressure sensor chip and/or the temperature sensor chip and/or the signal chain chip are COF encapsulated onto the flexible printed circuit by means of die Bonding and wire Bonding of bare chips;
or alternatively,
the absolute pressure sensor chip and/or the temperature sensor chip and/or the signal chain chip are COB encapsulated onto the micro circuit board by means of die Bonding and wire Bonding of bare chips.

15. The probe assembly according to any of claims 1 to 14, wherein the intracranial monitoring system is an artificial intelligence assisted intracranial monitoring system, and wherein the catheter is a neurophysiological monitoring catheter.

16. An artificial intelligence assisted intracranial monitoring system, wherein the artificial intelligence auxiliary intracranial monitoring system comprises:
the probe assembly according to any of claims 1 to 15, and
a monitor, the monitor comprising:
a display for displaying monitored parameters;
a pair of wireless communication modules configured to perform wireless digital communication between the probe assembly and the monitor, which comprises a first wireless communication module arranged on the probe assembly, and a second wireless communication module arranged on the monitor in pairing and in encrypted communication with the first wireless communication module; and
a host computer, which comprises at least a mainboard with a CPU, and an artificial intelligence-assisted prediction module integrated in the mainboard.

17. The artificial intelligence-assisted intracranial monitoring system according to claim 16, wherein the artificial intelligence-assisted intracranial monitoring system is configured to directly use the sensed data of the temperature sensor to perform temperature compensation calibration of a digital output signal of the probe assembly.

18. The artificial intelligence-assisted intracranial monitoring system according to claim 17, wherein the digital output signal goes through only one temperature compensation calibration before transmission to monitor.

19. The artificial intelligence-assisted intracranial monitoring system according to claim 16, wherein the artificial intelligence-assisted intracranial monitoring system further comprises accessories, the accessories including at least one of a multifunctional adapter, a puncture needle, and a subcutaneous tunnel type transfixion needle.

20. The artificial intelligence-assisted intracranial monitoring system according to claim 16, wherein the first wireless communication module and the second wireless communication module are Bluetooth communication modules.
